# EUROPEAN PATENT APPLICATION

(11) **EP 0 549 539 A1**
(43) Date of publication of application: **30.06.1993**
(21) Application number: 92830620.8
(22) Date of filing: 11.11.1992
(51) Int. Cl.: A61F 5/14, A43B 7/22

(54) **Kinesiological podoceptor plantar**

(30) Priority: 20.12.1991 IT BS910106 U
(71) Applicant: IL PODOLOGO S.r.l., I-25100 Brescia (IT)
(72) Inventor: Zucchini, Franco, I-2500 Muscoline, (Brescia) (IT)
(74) Representative: Manzoni, Alessandro

(57) **Abstract**

The invention concerns a kinesiological podoceptor including a plantar (11) , with a base surface presenting a layer of gripping material or adhesive substance (14) , and some ergoreceptors (12) presenting at least a surface suitable for restraining by pressure on any zone of the plantar base surface.

## Description

The present invention concerns a kinesiological podoceptor consisting of at least one plantar base and a series of ergoreceptors in various shapes and thicknesses which, applied to the plantar base, allow a quick and suitable personalization of the podoceptor.

An object of the present invention is to offer a kinesiological podoceptor plantar shaped so as to easily apply localized ergoreceptors, in a movable and interchangeable way, namely elements for the correction-stimulation in various shapes, dimensions and thicknesses as the kinesiologist will decide.

Another object of the invention is to provide a podoceptor kinesiologically plantar aimed, and in which the ergoreceptors can also be coupled, that is overlapped, in order to increase the thickness, or modified and decreased in shape and dimension simply by using a pair of scissors and with the advantage of allowing corection and modification to obtain the desired result.

A further object of the invention is to provide a kinesiological podoceptor plantar on which the ergoreceptors can be applied in any zone of the plantar base simply by pressing them, thus advantageously without sticking, sewing or injecting any material, and with the possibility of applying, shifting, or moving the elements of correction-stimulation with the same simplicity.

The enclosed drawing shows an embodiment of the invention, and exactly:
Fig.1 shows a plantar base where various-shaped ergoreceptors are applied; and
Fig.2 shows a cross view of an upside down plantar base at the level of ergoreceptors.

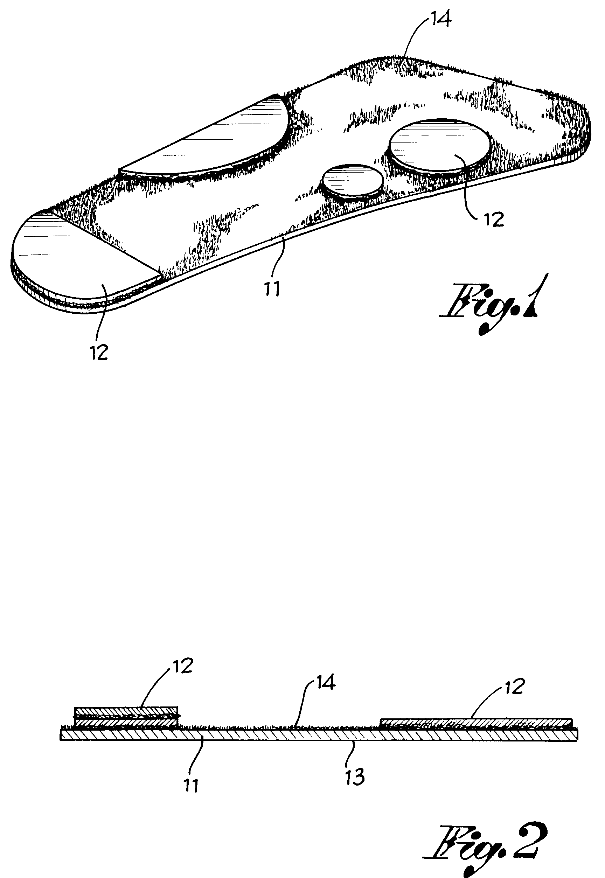

The proposed kinesiological podoceptor essentially consists of at least one plantar base 11 and a series of ergoreceptor elements 12.

The plantar base 11 can be supplied in any measure and size and made of convenient materials. The upper surface, which is intended to be in contact with the user's foot, presents a soft synthetic covering 13, for instance of a suede and waterrepellent sort to allow a perfect transpiration; the opposite surface, the lower one, presents a layer of adhesive material 14, preferably but not necessarily of the kind with little relief hooks, or adhesive on both the surfaces or like.

The ergoreceptors 12 can be in any shape and dimension, for instance in a textile material which can be cut and adapted as one wishes.

Each of them will have at least a suitable or treated surface so as to adhere and restrain to the lower surface of the plantar base, while the other side of the ergoreceptor may be neutral on adhesive grounds or, for what is of some ergoreceptors, also provided with adhesive material or substance for fixing the ergoreceptor elements in overlapping.

The plantar base is essentially suitable for receiveing and keeping, usually by simple pressure, the ergoreceptor elements on any zone of the lower surface in order to meet any requirement and the kinesiological tests. The ergoreceptors can be cut and then changed and/or modified and/or also overlapped in any subsequent moment in order to obtain the desired result.

## Claims

1. A kinesiological podoceptor plantar comprising at least one plantar base (11), which can be supplied in any measure and size, and a series of ergoreceptors (12), that is elements for correction-stimulation, characterized in that the plantar (11) has a base surface presenting a layer of gripping material or adhesive substance (14) and in that the ergoreceptors (12) have at least one surface suitable for restraining by preassure to any zone of the plantar base surface.

2. Kinesiological podoceptor plantar as claimed in claim 1, wherin the ergoreceptors (12) have any shape and dimension and can be applied in a movable and interchangeable way to the plantar base surface.

3. Kinesiological podoceptor as claimed in claims 1 and 2, wherin at least some of the ergoreceptors can be overlapped by adherence one another.
